# EUROPÄISCHE PATENTSCHRIFT

(11) **EP 0 910 312 B1**
(45) Veröffentlichungstag und Bekanntmachung des Hinweises auf die Patenterteilung: **17.12.2003**
(21) Anmeldenummer: 97930350.0
(22) Anmeldetag: 18.06.1997
(51) Int. Cl.: A61F 2/24

(54) **PROTHETISCHE HERZKLAPPE**
CARDIAC VALVE PROSTHESIS
VALVULE CARDIAQUE PROTHETIQUE

(30) Priorität: 24.06.1996 DE 19624951
(43) Veröffentlichungstag der Anmeldung: 28.04.1999
(73) Patentinhaber: ADIAM life science AG, 41812 Erkelenz (DE)
(72) Erfinder: JANSEN, Josef, D-50937 Köln (DE)
(74) Vertreter: Vomberg, Friedhelm, Dipl.-Phys.
(86) Internationale Anmeldenummer: DE9701299
(87) Internationale Veröffentlichungsnummer: WO97049357

(56) Entgegenhaltungen:
- WO-A-89/00841
- DE-B- 1 180 087
- US-A- 4 366 581

## Beschreibung

Die Erfindung betrifft eine prothetische Herzklappe, bestehend aus einem Stützgehäuse (Stent), an dem ein oder mehr flexible oder starre Segel und ein Nahtring befestigt sind. Gleichermaßen betrifft die Erfindung auch Bioprothesen oder mechanische Klappen mit einem Stützgehäuse, an dem ein Nahtring befestigt ist.

Die beispielsweise aus der DE 38 34 545 C2 bekannte Herzklappe besitzt ein Stützgehäuse aus einem zylindrischen Basisring, der durch drei auf dem Umfang jeweils um 120° versetzte, sich in axialer Richtung verengende Pfosten verlängert wird. Die zwischen den Pfosten liegenden Wandungsstirnflächen dienen zur Aufnahme und Befestigung von drei Segeln, welche das Schließen und Öffnen der Herzklappe gewährleisten. Im geschlossenen Zustand liegen die Segel an ihren freien Rändern unter Bildung eines Überlappungsbereiches aneinander. Neben den vorgenannten Aortaklappen sind auch sog. Mitralklappen bekannt, die anstelle von drei Segeln nur zwei Segel aufweisen.

Um die Herzklappe im Annulus gut anpassen und befestigen zu können, besitzen prothetische Herzklappen Nahtringe, die am Außenmantel des Stützgehäuses etwa in Höhe der Einströmebene befestigt werden. Die Nahtringe werden nach der Implantation mit dem Patientengewebe vernäht, um die prothetische Herzklappe fest in ihrer implantierten Lage zu verankern.

In der EP 0 443 993 B1 wird bereits ausgeführt, daß Nahtringe, die durch ein biokompatibles synthetisches Stoffgewebe überzogen oder vollständig hieraus gebildet sind, zwar den Vorteil besitzen, daß die Gleitfähigkeit für die Nadel und die Nahtfäden verbessert wird, was das Annähen im Annulus-Gewebe erleichtert, jedoch häufig den Nachteil besitzen, blutabsorbierend zu sein, was hämolytische oder thrombogene Auswirkungen haben kann. Abhilfe könnte ein flüssigkeitsabstoßendes Material wie z. B. Polytetrafluorethylen (Teflon) schaffen. Die Verwendung solcher flüssigkeitsabstoßenden Materialien steht jedoch mit der Forderung, daß die Außenbereiche des Nahtringes gut mit dem Patientengewebe verwachsen, im Widerspruch. Um diese unterschiedlichen Anforderungen erfüllen zu können soll nach der vorgenannten Patentschrift der Nahtring aus einem Verbundkörper aus zwei Materialien bestehen, von denen das erste im wesentlichen hämokompatibel ist, so daß es im wesentlichen kein Blut absorbiert und eine geringe hämölytische und thrombogene Wirkung hat, und dessen zweites Material im wesentlichen hydrophil porös ist, so daß es ein Einwachsen in das Patientengewebe fördert. Das erste Material ist hierbei jeweils stirnseitig des Nahtringes und das zweite Material an dem Umfangsmantel angeordnet. Nachteiligerweise ist der Aufbau eines solchen Nahtringes aufwendig und auch nur bedingt geeignet, bei auftretenden Scher- oder Torsionskräften genügend stabil zu sein.

In der EP 0 443 994 B1 wird ein Nahtring vorgeschlagen, der einen Kern besitzt, dessen innenseitige Randwulst in eine Nut am Stützgehäuseaußenmantel eingreift. Außerhalb dieses Eingriffsbereiches ist der Nahtring mit einem Überzug versehen, der so beschaffen sein soll, daß der Kontakt zwischen diesem Überzug und der Stützgehäuse-Außenmanteloberfläche einen genügend großen Widerstand gegen eine Relativdrehung des Nahtringes gegenüber dem Stützgehäuse gewährleistet. Auch dieser Nahtring besitzt die vorgenannten Nachteile.

Weitere Kern-Hülle-Strukturen des Nahtringes werden beispielsweise in der EP 0 119 357 A1, der EP 0 075 530 A1, der GB 2 021 420 A und der EP 0 145 166 A2 beschreiben. In der letztgenannten Druckschrift soll der Kernring aus einer Memorylegierung bestehen, die so ausgewählt und vorbehandelt ist, daß die körpereigene Temperatur des Patienten eine Kernringkontraktion bewirkt, die zu einer ausreichenden Klemmkraftwirkung führen soll.

Die DE-A 1180087, welche die Merkmale des Oberbegriffs von Anspruch 1 offenbart, beschreibt eine Herzklappenprothese mit einem Ventilkörper, einen mit Bezug auf den Ventilkörper beweglichen Ventilglied und einem der Befestigung der Prothese dienenden Fixierungsring, der als selbstständiges Bauteil ausgebildet und mit dem Ventilkörper verbindbar ist. Die Verbindung des Ventilkörpers und des Fixierungsringes kann entweder unlösbar sein, z.B. durch Verkleben oder Verschweißen oder lösbar ausgestaltet sein, z.B. in Form einer Form- oder Steckverbindung oder als Bajonettverschluß. Der von dem Ventilkörper abliegende Teil des Fixierungsringes ist mit einem grobmaschigen künstlichen Gewebe, beispielsweise gestricktem Polytetrafluorethylen, umhüllt, das zu einem lockeren Polster aufgerollt und mit Hilfe eines kräftigen Fadens zusammengehalten wird.

Es ist Aufgabe der vorliegenden Erfindung, eine prothetische Herzklappe der eingangs genannten Art anzugeben, deren Nahtring sicher an dem Stützgehäuse-Außenmantel befestigt ist, wobei der Nahtring weiterhin eine ausreichende Gleitfähigkeit für die beim Einnähen im Annulus notwendigen Nadeln und Fäden ebenso haben soll wie eine ausreichende Fexibilität. Das Nahtringgewebe soll schließlich geeignet sein, mit dem körpereigenen Gewebe des Patienten verwachsen zu können.

Diese Aufgabe wird durch die prothetische Herzklappe nach Anspruch 1 gelöst, die dadurch gekennzeichnet ist, daß der Nahtring an dem Stützgehäuse angeklebt ist, wobei der Nahtring, die Segel und die Außenoberfläche des Stützgehäuses aus demselben Material bestehen. Im Unterschied zu den bisher nach dem Stand der Technik bekannten Verbindungen, die auf Kraft- bzw. Reibungsschluß oder Formschluß oder Kombinationen hiervon beruhen, kann durch eine Klebverbindung nicht nur eine sicherere, dauerhaft haltbare Fixierung des Nahtringes an dem Herzklappen-Stützgehäuse erzielt werden, sondern auch ein einfacher Nahtring-Aufbau aus geeignetem bioverträglichem Material. Unter der ohne weiteres gewährleistbaren Voraussetzung, daß der verwendete Klebstoff nicht mit dem Patientengewebe oder dem Patientenblut in Berührung kommt, kann grundsätzlich auf sämtliche nach dem Stand der Technik bekannten Klebstoffe wie z. B. Epoxid-Harze zurückgegriffen werden.

Nach einer Weiterbildung der Erfindung wird jedoch als Klebstoff-Material dasselbe Material, vorzugsweise Polyurethan, verwendet, aus dem auch das Stützgehäuse, der Nahtring und die Segel bestehen. Damit kann die gesamte Herzklappe aus einem einheitlichen Werkstoff gefertigt werden, was insbesondere Materialspannungen in Folge unterschiedlicher thermischer Ausdehnungskoeffizienten (vor, während und nach dem Implantieren) wirkungsvoll vermeidet.

Nach einer weiteren Ausgestaltung der Erfindung ist die Klebstoffverbindung zwischen dem Nahtring und dem Stützgehäuse nach außen, d. h. an den Schnittkanten, versiegelt.

Der Nahtring, die Segel und das Stützgehäuse bestehen vorzugsweise aus einer mikroporösen, feinfibrillären Struktur, die ein Verwachsen mit dem körpereigenen Patientengewebe fördert. Falls notwendig oder wahlweise erwünscht, kann ein lokales Verwachsen mit körpereigenem Patientengewebe an beliebigen Stelles des Nahtringes durch Versiegelung (Kaschieren) der entsprechenden Stellen gezielt unterbunden werden, z.B. um das Einwachsen von Gewebe im Einströmquerschnitt zu verhindern.

Erfindungsgemäß gibt es zur Herstellung der vorbeschriebenen neuen Herzklappen mehrere Verfahren.

In einer ersten Verfahrensvariante wird ein Nahtringrohling durch Aufsprühen des Nahtringmaterials, das vorzugsweise aus Polyurethan besteht, auf eine Glasplatte oder einen Schlauchzylindermantel vorgefertigt. Dieser Nahtringrohling wird nach Ablösen von der Glasplatte oder dem Schlauchzylindermantel zu einer gewünschten Nahtring-Größe und Nahtring-Gestalt ausgeschnitten oder ausgestanzt, bevor der fertiggestellte Nahtring an dem Stützgehäuse aufgeklebt wird.

Das Aufkleben kann dadurch bewirkt werden, daß auf die vorgesehene Stützgehäusemantelaußenfläche Klebstoff aufgetragen wird, der Nahtring anschließend auf die mit Klebstoff beschichtete Oberfläche aufgeschoben, ausgerichtet und anschließend der Klebstoff getrocknet wird.

Alternativ hierzu ist es möglich, den vorgefertigten Nahtring auf die vorgesehene Stützgehäusefläche aufzuschieben und auszurichten, anschließend den Innenmantel des Nahtringes rundum des Stützgehäuses zumindest an einigen Stellen abzuheben und den Klebstoff in den Zwischenraum einzufüllen, bevor der Klebstoff getrocknet wird.

Ebenso besteht die Möglichkeit, den Klebstoff nach dem Aufschieben und Ausrichten des Nahtringes auf die Stützgehäuseoberfläche mittels einer Kanüle an die gemeinsame Anlagefläche des Nahtringes und des Stützgehäuses einzuspritzen und trocknen zu lassen. In einer hierzu möglichen Ausführungsvariante kann vor dem Aufschieben des Nahtringes die vorgesehene Stützgehäusefläche mit Klebstoff besprüht oder bestrichen werden, womit eine erste Klebstoffauflage geschaffen wird.

Eine alternative Herstellung der prothetischen Herzklappe ist im Anspruch 8 beschrieben, wonach der Nahtring unmittelbar in der gewünschten Breite und Dicke auf das Stützgehäuse aufgesprüht wird. Hierbei kann vorzugsweise um die Ringfläche, die für den Nahtringauftrag vorgesehen ist, das übrige Stützgehäuse beim Aufsprühen abgedeckt sein, so daß nur in den Bereichen das Stützgehäuse mit einem Nahtring-Auftrag versehen wird, an denen ein solcher Auftrag erwünscht ist. Hierzu kann das Abdeckmaterial für das übrige Stützgehäuse zumindest am Rand zu der für das Aufsprühen des Nahtringes freigelassenen Ringfläche eine Dicke aufweisen, die der gewünschten Nahtringdicke entspricht.

Erfindungsgemäß wählt man für Nahtring, Segel und Stützgehäuse dasselbe Material, insbesondere Polyurethan. Dadurch kann durch geeignete Prozeßführung eine stabile Vernetzung an der Verbindungsstelle des Nahtringes zum Stützgehäuse geschaffen werden, womit ein aus einem einheitlichen Material bestehender Körper mit einfachem Nahtring-Aufbau geschaffen werden kann.

Ausführungsbeispiele der Erfindung sind in den Figuren dargestellt. Es zeigen
- Figur 1: eine perspektivische Ansicht einer drei-segeligen Aorta-Klappe (ohne Nahtring),
- Figur 2: einen Schnitt entlang der Ebene A-A in Figur 1,
- Figur 3: eine perspektivische Ansicht einer zwei-segeligen Mitral-Klappe (ohne Nahtring),
- Figur 4: einen Schnitt entlang der Linie B-B in Figur 3,
- Figur 5: eine perspektivische Ansicht einer weiteren Mitralklappe und
- Figur 6: eine Draufsicht auf die Mitralklappe nach Fig. 5, jeweils ohne Nahtring.

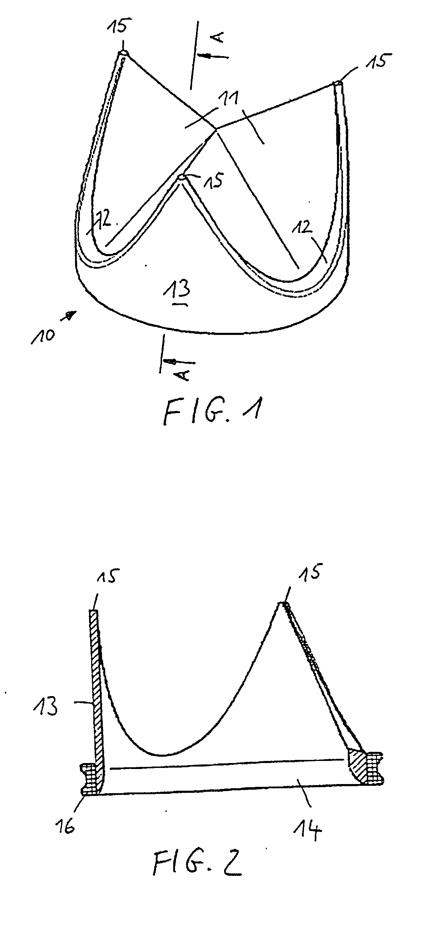

Die in Figuren 1 und 2 dargestellte Herzklappe besteht im wesentlichen aus einem Stützgehäuse 10 sowie drei Segeln 11, die an radial nach außen gestellten Schrägflächen, nämlich den Stirnflächen 12 der Wandung 13 des Stützgehäuses befestigt sind. Das Stützgehäuse 10 besitzt einen Basisring 14, dessen Durchmesser den Klappendurchfluß bestimmt und an dessen Einlaufbereich die Innenmantelkanten abgerundet sind. Die Wandung 13 ist im wesentlichen senkrecht zu der Basisringgrundfläche angeordnet, wobei drei Spitzen 15 als Pfosten in einem Winkelabstand von 120° zueinander herausragen, zwischen denen die Stirnfläche 12 der Wandung 13 kreisbogenförmig verläuft. Die Innenkante dieser Stirnflächen 12 zwischen zwei Pfosten liegt jeweils in einer Ebene, die sich als Schnittlinie eines Zylinders mit einer schräg zu dessen Längsachse angeordneter Ebene ergibt. Hierdurch ergibt sich ein Anstellwinkel der drei Ebenen von ca. 80° relativ zur Basisringgrundfläche. Das Stützgehäuse besteht aus elastischem Kunststoff. Es kann sich hierbei um ein Thermoplast wie Polyamid handeln, das beispielsweise durch Spritzgießen hergestellt und nach Aufrauhung der Oberfläche mit Polyurethan beschichtet worden ist. Die Segel 11 bestehen aus einer flexiblen, dünnen Kunststoffolie, die vorzugsweise aus Polyurethan gefertigt ist.

Die Wandung 13 ist außen glattflächig ausgefüllt und dient, wie aus Figur 2 ersichtlich, zur Befestigung eines Nahtringes 16, der eine im wesentlichen hohlzylindrische Form hat, deren Außenmantel an den beiden Stirnkanten ringförmige Wülste aufweist. Diese Nahtringmaterial besteht ebenfalls aus Polyurethan.

Zur Fertigung der dargestellten Aorta-Klappe wird das durch Spritzgießen hergestellte Stützgehäuse 10 zunächst mit den drei Segeln 11 versehen, die an den Stirnflächen 12 verklebt werden. Die Segel selbst sind zuvor aus einer ebenen Polyurethan-Folie ausgeschnitten worden. Anschließend wird entweder der bereits vorgefertigte Nahtring im Bereich des Basisringes 14 an dem Stützgehäuseaußenmantel angeklebt oder unmittelbar aufgesprüht. Im erstgenannten Fall wird der Nahtring zunächst auf einer Glasplatte oder schlauchförmig auf einem Zylinderkörper aufgesprüht, wodurch Vliese mit mikroporöser feinfibrillärer Struktur hergestellt werden. Nach dem Ausschneiden des vorgefertigten Teils in die gewünschte Nahtringform und dem Versiegeln etwaiger Schnittkanten wird der Nahtring auf den Außenmantel des Stützgehäuses 10 in Höhe des Basisringes 14 aufgeschoben und ausgerichtet und anschließend zwischen dem Nahtring und dem Stützgehäuse 10 eingespritzt. Alternativ oder zusätzlich kann zuvor der als Trägermantelfläche vorgesehene Bereich vor dem Aufschieben und Ausrichten des Nahtringes 16 mit Klebstoff beschichtet werden. In einer abschließenden Behandlung wird stromaufseitig und stromabseitig die Übergangszone des Nahtringes 16 zum Außenmantel des Stützgehäuses 10 ringförmig mit Klebstoff versiegelt.

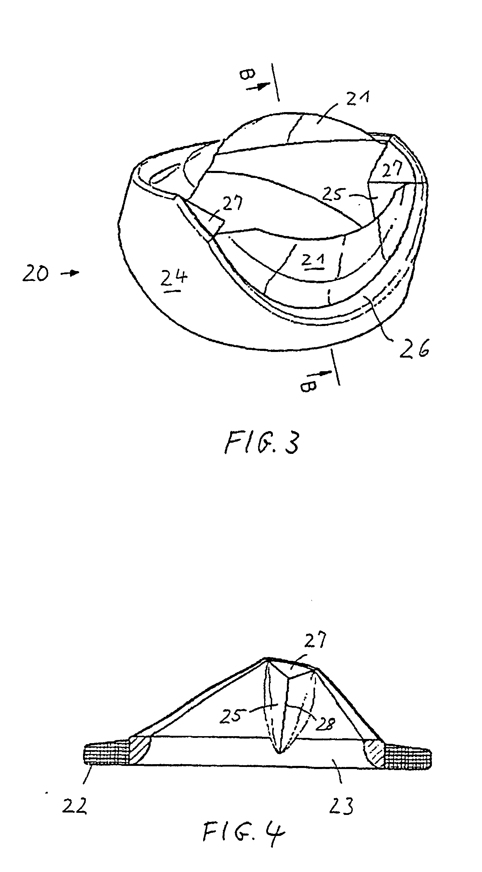

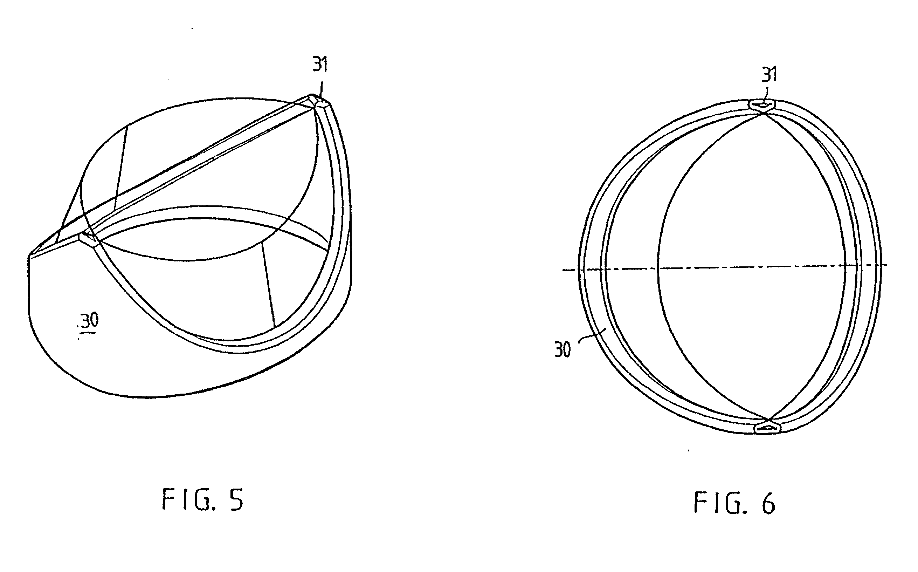

Die in Figuren 3 und 4 dargestellte Mitral-Herzklappe besteht aus einem Stützgehäuse 20 mit zwei Segeln 21 und einem Nahtring 22, der außenmantelseitig in Höhe des Einlaufbereiches des Stützgehäuses 20 befestigt ist. Das einstückige Stützgehäuse 20 weist einen Basisring 23 auf, dessen Innenkanten im Einlaufbereich in nach dem Stand der Technik bekannterweise abgerundet sind. Die im Wesentlichen zur Basisringgrundfläche senkrechte Wandung 24 teilt sich in eine erste Wandung mit geringerer Krümmung und eine zweite Wandung mit größerer Krümmung auf, die zusammen in Draufsicht auf die Basisgrundfläche betrachtet zwei Halbellipsen mit einer gemeinsamen Längsachse bilden. Demgemäß sind die halben Querachsen unterschiedlich lang, vorzugsweise beträgt ihr Längenverhältnis 1:2. Die obere Stirnfläche 26 der Wandung 24 dient als Befestigungsfläche für die Segel 21. Jede der beiden Stirnflächen 26 zwischen zwei Pfosten 25 verläuft mit ihrer Innenkante in einer schräg zur Basisringgrundfläche, die den Einlaufbereich darstellt, liegt. Im vorliegenden Fall liegt die Verbindungslinie der Segel 21 mit der oberen Innenkante der Wandungen 24 jeweils in einer Ebene, die bezogen auf die Basisringgrundfläche um einen Winkel von ca. 50° bzw. von 35° verläuft. Die Segelanstellung ist somit unterschiedlich. Die Pfosten 25 besitzen eine prismatische Kontur und verbreitern sich zu ihren oberen Stirnflächen 27, die im wesentlichen dreieckförmig ausgeführt sind, gleichmäßig. In einer Draufsicht auf die Innenwandung des Stützgehäuses 10 (siehe Figur 4) betrachtet sind die Pfosten V-förmig ausgeführt und enden keilförmig oberhalb der Basisring-Grundfläche im Einlaufbereich des Stützgehäuses 10. Die Querschnitte der Pfosten 25 parallel zur Basisring-Grundfläche bilden im wesentlichen gleichschenklige ähnliche Dreiecke, wobei die Pfostenlängsachse 28 bezogen auf die Basisring-Grundfläche nicht vertikal, sondern gegenüber einer Flächennormalen leicht geneigt, beispielsweise unter einem Winkel von 15° verläuft. Eine dementsprechende Neigung besitzt auch die Stirnfläche 27 der Pfosten gegenüber der Basisring-Grundfläche. Die Pfosten 25 ersetzen die bei natürlichen Herzklappen gegebenen Kommissurenbereiche und dienen mit ihren etwa gleichlangen Dreiecksschenkeln als prismatische Innenauflage für die Segel 21.

Eine weitere Form der Mitralklappe ist in Fig. 5 dargestellt. Im Unterschied zu der in Fig. 3 und 4 dargestellten Mitralklappe ist der Pfosten in die Wandung 30 dickengleich integriert. Im Bereich der vorbeschriebenen Pfosten hat die Wandung die größte Höhe - gemessen vom Basisring - und endet entweder spitzwinklig oder, wie in Fig. 5 und 6 dargestellt, in einer Abflachung 31. Der Pfosten tritt somit nicht mehr geometrisch "aus der Wandung 30" heraus. Die Wandung 30 ist zwischen den beiden Pfostenbereichen dicker als im Bereich der Pfosten (siehe Bezugszeichen 31).

Im Unterschied zu dem in Figur 2 dargestellten Nahtring ist der Nahtring 22 nach Figur 4 aus anatomischen Gründen zur Reduzierung der Nahtspannungen breitrandig in Form einer Nahtkappe ausgeführt. Diese sog. supra-annuläre Bauform wird bei Mitral-Herzklappen bevorzugt angewendet. Für die Befestigung des Nahtringes 22 gilt entsprechendes wie zuvor zu der Aorta-Klappe beschrieben.

Die Vorteile der verwendeten vliesartigen Nahtringe aus Polyurethan liegen in der sehr guten chirurgischen Handhabbarkeit im Hinblick auf die Gleitfähigkeit des Materials beim Vernähen. Gleichermaßen ist es möglich, den bereits an dem Stützgehäuse befestigten Nahtring noch vor der Implantation durch Schneiden zu bearbeiten, um eine bessere Passgenauigkeit im Annulus des Patienten zu gewährleisten. Die Polyurethan-Nahtringe besitzen eine gute Ausreißfestigkeit und eine hohe Elastizität. Ferner zeichnen sich Polyurethan-Nahtringe durch eine gute Hämokompatibilität aus. Aufgrund der mikrofibrillären Struktur ist ein schnelleres und vor allem kontrollierteres Einheilen mit umliegenden Patientengewebe möglich. Es bildet sich nur eine sehr dünne Neointima, eine Bildung von Riesenzellen und übermäßigem Pannus wird vermieden. Thromboembolische Komplikationen werden ebenso reduziert wie das Risiko perivalvulärer Leaks und einer Endokarditis. Die Gewebeverträglichkeit des Nahtringes ist gut.

Ein weiterer Vorteil liegt darin, daß die gesamte Klappe an ihrer Oberfläche aus gleichem Material besteht. Die Fertigung bzw. Anbindung des Nahtringes am Stent wird vereinfacht und auf eine geringere Anzahl von Arbeitsschritten beschränkt. Eine geringere Anzahl von erforderlichen Bauteilen sowie das elastische Gewebe des Nahtringes führen zu einer sicheren Fixierung des Nahtringes im gesamten Anlagebereich dieses Nahtringes an dem Stützgehäuse. das erfindungsgemäße Verfahren ermöglicht die von der eigentlichen Prothesenfertigung (Fertigung des Stützgehäuses mit Segel) getrennte und weniger aufwendige Herstellung des Nahtringes und ein anschließendes, relativ einfaches Zusammenfügen des Nahtringes mit dem Stützgehäuse durch kleben.

Bei der Fertigung der beschriebenen Herzklappe können die jeweils gefertigten Segel durch Kleben oder Verschweißen an den Stirnflächen des Stützgehäuses befestigt werden. Alternativ hierzu ist es auch möglich, die Herzklappe mittels der nach dem Stand der Technik bekannten Spritzgußtechnik einschließlich des 2-Komponentenspritzens herzustellen, bei dem zunächst das Stützgehäuse gefertigt und anschließend hieran die Segel durch Spritzgießen angebracht werden. Eine weitere Möglichkeit ist die Anwendung der sogenannten Tauchtechnik. Hierzu wird das z.B. aus Polyamid befestehende Stützgehäuse nach einer Beschichtung mit Polyurethan auf einen entsprechenden Tauchdorn mit Formflächen für die Segel aufgeschoben und anschließend der Tauchdorn mit dem Stützgehäuse in eine flüssige Kunststofflösung (Polyurethan) eingetaucht und so lange taumelnd bewegt, bis die gewünschte Dickenverteilung erreicht ist. Während des Taumelns härtet der Kunststoff aus.

An die derart gefertigte Einheit aus dem Stützgehäuse und dem Segel oder den Segeln wird abschließend der Nahtring in der oben beschriebenen Weise befestigt.

## Patentansprüche

1. Prothetische Herzklappe, bestehend aus einem Stützgehäuse (10, 20), an dem ein oder mehr flexible oder starre Segel (11, 21) und ein Nahtring (16, 23) befestigt sind, **dadurch gekennzeichnet, dass** der Nahtring (16, 22) an dem Stützgehäuse (10, 20) angeklebt ist, wobei der Nahtring, das oder die Segel und die Außenoberfläche des Stützgehäuses aus demselben Material bestehen.

2. Herzklappe nach Anspruch 1 , **dadurch gekennzeichnet, dass** der Nahtring (16, 22), die Segel (11, 21), die Außenoberfläche des Stützgehäuses (10, 20) und der Klebstoff aus demselben Material, vorzugsweise Polyurethan bestehen und/oder dass die Klebstoffverbindung zwischen dem Nahtring (16, 22) und dem Stützgehäuse (10, 20) nach außen versiegelt ist.

3. Herzklappe nach einem der Ansprüche 1 oder 2, **dadurch gekennzeichnet, dass** der Nahtring (16, 22) eine mikroporöse, feinfibrilläre Struktur aufweist.

4. Verfahren zur Herstellung einer prothetischen Herzklappe, bei der der Nahtring, die Außenoberfläche des Stützgehäuses und die Segel aus demselben Material bestehen, nach einem der Ansprüche 1 bis 3, wobei ein Nahtring-Rohling durch Aufsprühen des Nahtringmaterials, vorzugsweise Polyurethan, auf eine im wesentlichen ebene Fläche wie eine Glasplatte oder einen Schlauchzylindermantel vorgefertigt und anschließend der Nahtring-Rohling zu einem Nahtring (16, 22) gewünschter Größe ausgeschnitten oder ausgestanzt und abschließend auf das Stützgehäuse aufgeklebt wird, bevor die Segel befestigt werden.

5. Verfahren nach Anspruch 4, **dadurch gekennzeichnet, dass** auf die vorgesehene Stützgehäusefläche Klebstoff aufgetragen und der Nahtring (16, 22) auf die mit Klebstoff beschichtete Stützgehäusefläche aufgeschoben, ausgerichtet und anschließend der Klebstoff getrocknet wird.

6. Verfahren nach Anspruch 4, **dadurch gekennzeichnet, dass** der vorgefertigte Nahtring (16, 22) auf die vorgesehene Stützgehäusefläche aufgeschoben und ausgerichtet wird, anschließend der Innenmantel des Nahtringes rundum des Stützgehäuses (10, 20) abgehoben und Klebstoff in den Zwischenraum eingefüllt wird und abschließend der Klebstoff getrocknet wird.

7. Verfahren nach Anspruch 4, **dadurch gekennzeichnet, dass** der vorgefertigte Nahtring auf die vorgesehene Stützghäusefläche aufgeschoben und ausgerichtet wird, anschließend der Klebstoff mittels einer Kanüle an die gemeinsame Anlagefläche des Nahtringes (16, 22) und des Stützgehäuses (10, 20) eingespritzt und abschließend der Klebstoff getrocknet wird, wobei vorzugsweise vor dem Aufschieben des Nahtringes (16, 22) die vorgesehene Stützgehäusefläche mit dem Klebstoff besprüht und hiermit eine erste Klebstofflschicht aufgetragen wird.

8. Verfahren zur Herstellung einer prothetischen Herzklappe nach einem der Ansprüche 1 bis 3, bei der der Nahtring, die Außenoberfläche des Stützgehäuses und die Segel aus demselben Material bestehen, wobei der Nahtring unmittelbar in der gewünschten Breite und Dicke auf das Stützgehäuse aufgesprüht wird, bevor die Segel befestigt werden.

9. Verfahren nach Anspruch 8, **dadurch gekennzeichnet, dass** um die für den Nahtringauftrag vorgesehene Ringfläche das übrige Stützgehäuse beim Aufsprühen abgedeckt wird, wobei vorzugsweise das Abdeckmaterial für das übrige Stützgehäuse zumindest am Rand zu der für das Aufsprühen des Nahtringes freigelassenen Ringfläche eine Dicke aufweist, die der gewünschten Nahtringdicke entspricht.

10. Verfahren nach einem der Ansprüche 4 bis 9, **dadurch gekennzeichnet, dass** das Stützgehäuse, die Segel und/oder der Nahtring aus Polyurethan bestehen.

## Claims

1. A prosthetic heart valve comprised of a support housing (10, 20) to which one or more flexible or rigid leaflets (11, 21) and a suture ring (16, 23) are fastened, **characterised in that** the suture ring (16, 22) is glued to the support housing (10, 20), whereby the suture ring, the leaflet or the leaflets and the outer surface of the support housing are composed of the same material.

2. A heart valve according to claim 1, **characterised in that** the suture ring (16, 22) the leaflets (11, 21), the outer surfaces of the support housing (10, 20) and the adhesive are comprised of the same material, preferably polyurethane and/or that the adhesive bond between the suture ring (16, 22) and the support housing (10, 20) is sealed toward the exterior.

3. A heart valve according to claims 1 to 2, **characterised in that** the suture ring (16, 22) has a microporous, fine fibrillar structure.

4. Method of producing a prosthetic heart valve comprising a suture ring, an outer surface of the support housing and the leaflets composed of the same material, according to one of the claims 1 to 3, wherein a suture ring blank is prefabricated by spraying the suture ring material, preferably polyurethane, upon a substantially planar surface like a glass plate or on a hose cylinder shell and then the suture ring blank is cut or stamped to a suture ring (16, 22) of the desired size and finally, before the leaflets are fastened, the suture ring (16, 22) is glued to the support housing (10, 20).

5. Method according to claim 4, **characterised in that** adhesive is coated onto the support housing surfaces which are provided and the suture ring (16, 22) is shoved onto the support housing surfaces which is coated with adhesive, aligned and the adhesive is then dried.

6. Method according to claim 4, **characterised in that** the prefabricated suture ring (16, 22) is shoved and aligned onto the support housing surfaces which are provided and is aligned then the inner wall of the suture ring is raised around the support housing (10, 20) and adhesive is filled into the intervening space and then the adhesive is dried.

7. Method according to claim 4, **characterised in that** the prefabricated suture ring is shoved an aligned onto the support housing surfaces which are provided, the adhesive then being injected by means of a cannula on the common abutment surfaces of the suture ring (16, 22) and the support housing and the adhesive is then dried, whereby preferably before the suture ring (16, 22) is shoved onto the support housing surfaces which are provided, the latter are sprayed with the adhesive and a first adhesive layer is thus applied.

8. Method for producing a prosthetic heart valve according to claims 1 through 3, wherein the suture ring, the outer surface of the support housing and the leaflets are composed of the same material, whereby the suture ring is injected directly in the desired width and thickness on the support housing, before the leaflets are fastened.

9. Method according to claim 8, **characterised in that** the ring surfaces remaining beyond those provided for application of the suture ring are masked in the act of spraying, whereby preferably the masking material for the remainder of the support housing at least at the edge of the annular surface which is free to receive the suture ring, has a thickness which corresponds to the desired suture ring thickness.

10. Method according to one of the claims 4 to 9, **characterised in that** the support housing, the leaflets and/or the suture ring are composed of polyurethane.

## Revendications

1. Valvule cardiaque prothétique se composant d'un boîtier de support (10, 20) sur lequel sont fixés un ou plusieurs voiles flexibles ou rigides (11, 21) et un anneau de suture (16, 22), **caractérisée par le fait que** l'anneau de suture (16, 22) est collé sur le boîtier de support (10, 20), l'anneau de suture, le ou les voile(s) et la surface extérieure du boîtier de support étant composés de la même matière.

2. Valvule cardiaque selon la revendication 1, **caractérisée par le fait que** l'anneau de suture (16, 22), les voiles (11, 21), la surface extérieure du boîtier de support (10, 20) et la colle se composent de la même matière, de préférence de polyuréthane, et/ou que l'assemblage à colle entre l'anneau de suture (16, 22) et le boîtier de support (10, 20) est scellé vers l'extérieur.

3. Valvule cardiaque selon l'une des revendications 1 ou 2, **caractérisée par le fait que** l'anneau de suture (16, 22) présente une structure microporeuse à fibrilles fines.

4. Procédé de fabrication d'une valvule cardiaque prothétique dans laquelle l'anneau de suture, la surface extérieure du boîtier de support et les voiles sont composés de la même matière, selon l'une des revendications 1 à 3, une ébauche d'anneau de suture étant préfabriquée en appliquant par pulvérisation la matière de l'anneau de suture, de préférence du polyuréthane, sur une surface pour l'essentiel plane, telle qu'une plaque de verre ou une enveloppe d'un cylindre en tuyau flexible, et, ensuite, l'ébauche d'anneau de suture étant découpée ou poinçonnée de manière à obtenir un anneau de suture (16, 22) de grandeur souhaitée et, finalement, ce dernier étant collé sur le boîtier de support avant que les voiles soient fixés.

5. Procédé selon la revendication 4, **caractérisé par le fait que** l'on applique de la colle sur la surface prévue du boîtier de support et que l'on fait glisser l'anneau de suture (16, 22) sur la surface du boîtier de support, qui est revêtue de colle, on l'ajuste et que, ensuite, la colle est séchée.

6. Procédé selon la revendication 4, **caractérisé par le fait que** l'on fait glisser l'anneau préfabriqué de suture (16, 22) sur la surface prévue du boîtier de support et on l'ajuste, que, ensuite, la paroi intérieure de l'anneau de suture est soulevée tout autour du boîtier de support (10, 20) et on introduit de la colle dans l'intervalle et que, finalement, la colle est séchée.

7. Procédé selon la revendication 4, **caractérisé par le fait que** l'on fait glisser l'anneau préfabriqué de suture sur la surface prévue du boîtier de support et on l'ajuste, que, ensuite, la colle est injectée au moyen d'une canule sur la surface commune d'appui de l'anneau de suture (16, 22) et du boîtier de support (10, 20) et que, finalement, la colle est séchée, de préférence, avant que l'on fasse glisser l'anneau de suture (16, 22) là-dessus, la surface prévue du boîtier de support étant aspergée de la colle et, ainsi, une première couche de colle étant appliquée.

8. Procédé de fabrication d'une valvule cardiaque prothétique selon l'une des revendications 1 à 3, dans laquelle l'anneau de suture, la surface extérieure du boîtier de support et les voiles sont composés de la même matière, l'anneau de suture étant appliqué par pulvérisation directement dans la largeur et l'épaisseur souhaitées sur le boîtier de support avant que les voiles soient fixés.

9. Procédé selon la revendication 8, **caractérisé par le fait que**, autour de la surface annulaire prévue pour l'application de l'anneau de suture, le reste du boîtier de support est recouvert lors de l'application par pulvérisation, de préférence la matière de recouvrement pour le reste du boîtier de support présentant - au moins sur le bord vers la surface annulaire laissée libre pour l'application par pulvérisation de l'anneau de suture - une épaisseur qui correspond à l'épaisseur souhaitée de l'anneau de suture.

10. Procédé selon l'une des revendications 4 à 9, **caractérisé par le fait que** le boîtier de support, les voiles et/ou l'anneau de suture sont composés de polyuréthane.
